# EUROPEAN PATENT SPECIFICATION

(11) **EP 3 721 864 B1**
(45) Date of publication and mention of the grant of the patent: **10.07.2024**
(21) Application number: 18887162.8
(22) Date of filing: 05.07.2018
(51) Int. Cl.: A61K 8/60, A61K 8/44, A61K 8/49, A61K 8/41, A61K 8/73, A61Q 19/00

(54) **COSMETIC COMPOSITION FOR PROMOTING SKIN EXFOLIATION**
KOSMETISCHE ZUSAMMENSETZUNG ZUR FÖRDERUNG DER HAUTABSCHÄLUNG
COMPOSITION COSMÉTIQUE DESTINÉE À FAVORISER L'EXFOLIATION DE LA PEAU

(30) Priority: 04.12.2017 KR 20170165268
(43) Date of publication of application: 14.10.2020
(73) Proprietor: LG Household & Health Care Ltd., Seoul 03184 (KR)
(72) Inventor: AHN, Byung-Jun, Daejeon 34114 (KR); LEE, Seol-Hoon, Daejeon 34114 (KR); KANG, Nae-Gyu, Daejeon 34114 (KR); GOH, Areum, Daejeon 34114 (KR); KIM, Jin-Hyun, Daejeon 34114 (KR)
(74) Representative: Goddar, Heinz J.
(86) International application number: PCT/KR2018/007659
(87) International publication number: WO 2019/112129

(56) References cited:
- IT-A1- MI20 110 643
- JP-A- H10 245 331
- KR-A- 20040 043 596
- KR-A- 20100 061 881
- KR-A- 20100 061 881
- KR-A- 20130 090 169
- KR-A- 20150 049 801
- KR-B1- 100 678 864
- DATABASE GNPD [online] MINTEL; 31 March 2014 (2014-03-31), ANONYMOUS: "Invisible Peeling Booster", XP055830524, retrieved from https://www.gnpd.com/sinatra/recordpage/2375677/ Database accession no. 2375677
- DATABASE GNPD [online] MINTEL; 28 June 2016 (2016-06-28), ANONYMOUS: "Clarifying Cleanser", XP055830540, retrieved from https://www.gnpd.com/sinatra/recordpage/4013023/ Database accession no. 4013023
- DATABASE GNPD [online] MINTEL; 2 April 2009 (2009-04-02), ANONYMOUS: "The Body Refiner", XP055830541, retrieved from https://www.gnpd.com/sinatra/recordpage/1084210/ Database accession no. 1084210
- DATABASE GNPD [online] MINTEL; 20 May 2008 (2008-05-20), ANONYMOUS: "Peeling Cream", XP055830543, retrieved from https://www.gnpd.com/sinatra/recordpage/915200/ Database accession no. 915200
- DATABASE GNPD [online] MINTEL; 5 September 2017 (2017-09-05), ANONYMOUS: "Slimming and Firming Anti-Cellulite Lotion", XP055830544, retrieved from https://www.gnpd.com/sinatra/recordpage/5070295/ Database accession no. 5070295
- ANON: "Bamboo: Sensocel", 31 August 2017 (2017-08-31), XP055833599, Retrieved from the Internet <URL:https://www.cossma.com/fileadmin/all/cossma/Archiv/2017/07_08/CSDE1707_27_CFF.pdf> [retrieved on 20210820]

## Description

### [TECHNICAL FIELD]

The present disclosure relates to a cosmetic use of a cosmetic composition for promoting skin exfoliation.

### [BACKGROUND ART]

In general, skin is divided into an epidermal layer and a dermal layer, of which epidermal layer is divided into a stratum corneum layer, a granular layer, a stratum spinosum layer and a basal layer. Skin cells produced through the basal layer take a form of thin and wide cells due to the denuclearization process of cells and changes in the lipid bilayer to the stratum corneum layer, and when they reach the stratum corneum, they become to have a form of dead cells filled with hydrophobic keratin proteins inside. In addition, between keratinocytes of the stratum corneum, a lamella layer composed of ceramides as a main component is formed, and a structure bound to the corneodesmosome between keratinocytes is taken. The stratum corneum composed of keratinocytes and ceramides shows a protective effect from foreign substances, and at the same time, it functions as a protective film that can prevent moisture from escaping, and the like. A normal stratum corneum has a thickness of about 15-20 layers, and the action of serine protease activated outside the stratum corneum causes the decomposition of corneodesmosome and eventually undergoes a process of complete detachment from the skin. This process takes about 15-20 days for normal cells. (Britsh J. of Dermatology, 86, 14-19, 1974; K.M.Halprin and Cosmetical Bulletin, 12(4), 265-271, 1988; M.Takahash) Even if it is not a genetic disease such as ichthyosis, aging, dryness, and acne skin have a longer period of the above exfoliation process than normal, and therefore the stratum corneum stratification which thickens the stratum corneum layer appears.

It is known that the stratum corneum stratification is generally caused by factors such as decreased skin moisturizing ability, reduced expression of serine protease or decreased activity, ultraviolet and decreased cellular activity, and the like. In order to eliminate the stratum corneum stratification produced in this way, generally, methods such as exfoliation by a physical scrub preparation, exfoliation by a chemical preparation, and physiological cell activity increase are used. These types of exfoliation methods are intended to solve external problems such as roughness or dullness of skin by artificially removing external keratinocytes and through replacement of the outermost stratum corneum layer with new keratinocytes from inside, resolving the stratum corneum stratification.

Among methods for resolving the stratum corneum stratification, in particular, the method using AHA (Alpha hydroxy acid) of chemical exfoliation methods is representative, and in addition, BHA (beta hydroxy acid) and PHA (poly hydroxy acid) are also representative methods. The chemical exfoliation method has additional effects such as removing fine wrinkles, removing blemishes, improving rough skin, and the like, through exfoliation. AHA used for chemical exfoliation may have an effect by treating skin at a high concentration (30 - 70 %) for a short time (1 - 15 minutes) (Clinical, Cosmetic and Investigational Dermatology, 6, 281-288, 2013; J. Sharad), but it also shows effects such as slowly exfoliating the stratum corneum when used at a concentration of 10% or less (J.AM.Acad.Dermatol., 11, 867-879, 1984; Van Scott), enhancing skin moisturizing (happi, jully, 66-68, 1994), alleviating fine wrinkles (Cutis, 43, 222-228, 1989; Van Scott), and the like, and therefore it is used for cosmetics, and the like. The action mode of AHA is inserted into the stratum corneum and releases hydrogen ions to weaken corneodesmosome binding and lead to exfoliation, and when applied at a low pH, the insertion of the stratum corneum is smooth and the delivery of hydrogen ions is high, so inevitably low pH is required. Therefore, as low pH causes side effects such as skin stinging, itching, erythema, and the like, research to overcome these side effects has been conducted. However, when pH is adjusted or chemical modification is performed to reduce side effects, the efficacy is offset, and thus a need for a new exfoliation material that can exhibit exfoliation efficacy such as AHA even in weak acidity or neutrality has emerged.

As conventional efforts to achieve this efficacy, attempts have been made to reduce irritation and maintain or increase the exfoliation efficacy by slowing the skin permeation rate due to large molecular weight using PHA (Polyhydroxy acid) having a hydroxyl group and a carboxyl group (Korean Registered Patent No. 10-1388052). However, even in this case, when the pH is raised, its efficacy is rapidly lost, and therefore it should be used in an acidic condition, and thus there is still a potential for irritate the skin.

Conventional chemical exfoliating agents such as AHA or PHA exhibit their effects based on weakening of binding protein between keratinocytes due to delivery of hydrogen ions through low pH. Accordingly, when the pH is adjusted to a weakly acidic-neutral pH in order to alleviate side effects caused by skin irritation, they have a problem that their activities are reduced by 80% or more, so it is difficult to provide an advantage of substantially exfoliation.

KR 2010 0061 881 A discloses exfoliating compositions which comprise functionally defined components comprising a calcium-binding substance and a substance that changes protein structure. Respective cosmetic compositions are also known from Mintel Database, Database accession no. 2375677; Mintel Database, Database accession no. 4013023; Mintel Database, Database accession no. 1084210; Mintel Database, Database accession no. 915200; or Mintel Database, Database accession no. 5070295.

### [DISCLOSURE]

### [TECHNICAL PROBLEM]

The present disclosure is to solve the aforementioned problems, and is to provide a cosmetic use of a cosmetic composition with an excellent exfoliation effect than the efficacy of the conventional effective raw material even at a weakly acidic-neutral pH and low skin irritation.

### [TECHNICAL SOLUTION]

To achieve the aforementioned objects, the present disclosure provides a cosmetic use of a cosmetic composition comprising a combination of at least one of serine and carnitine and at least one of allantoin and creatine for promoting skin exfoliation.
Further embodiments are disclosed in the dependent claims.

The at least one of allantoin and creatine may further comprise at least one selected from urea and hydroxyethyl urea.

According to one aspect of the present disclosure, the cosmetic composition for promoting skin exfoliation may be characterized by further comprising a polymer material.

According to one aspect of the present disclosure, the polymer material of the cosmetic composition for promoting skin exfoliation may be characterized by at least one selected from the group consisting of cellulose nanofiber, carboxymethyl cellulose (CMC), hyaluronic acid and carbomer.

According to one aspect of the present disclosure, the cosmetic composition for promoting skin exfoliation is characterized by pH 5 to 7.5.

According to one aspect of the present disclosure, the content of the at least one of serine and carnitine in the composition is characterized by 0.001 to 20 % by weight based on the total composition weight.

According to one aspect of the present disclosure, the content of the at least one of allantoin and creatine in the composition is characterized by 0.001 to 10 % by weight based on the total composition weight.

According to one aspect of the present disclosure, the content of the polymer material in the composition is characterized by 0.0001 to 5 % by weight based on the total composition weight.

### [ADVANTAGEOUS EFFECTS]

The cosmetic composition may have an excellent exfoliation effect compared to the conventional effective raw material even at a weakly acidic-neutral pH and also exhibit low skin irritation.

In particular, the present disclosure maximizes exfoliating ability through a combination of raw materials having a synergistic effect, thereby exhibiting an effect of increasing efficacy and reducing side effects which are unique side effects of a raw material that may be caused when using one kind of raw material at a high concentration.

### [BRIEF DECRIPTION OF THE DRAWINGS]

FIG. 1 is the result of the experiment confirming the exfoliating ability by the concentration of gluconolactone and pH.
FIG. 2 is the result of the experiment confirming the exfoliating ability by the concentration of allantoin and polymer material alone.
FIG. 3 is the result of the experiment confirming the exfoliating ability of the combination by the concentration of allantoin and cellulose nanofiber.
FIG. 4 is the result of the experiment confirming the exfoliating ability by the concentration of carnitine, creatine and carnosine alone.
FIG. 5 is the result of the experiment confirming the exfoliating ability of the combination by the concentration of carnitine, creatine, allantoin and carnosine.
FIG. 6 is the result of the experiment confirming the exfoliating ability by the concentration of serine.
FIG. 7 is the result of the experiment confirming the exfoliating ability by the concentration of the combination of serine, creatine and allantoin.
FIG. 8 is the result of the experiment confirming the exfoliating ability of various combination examples.

### [MODE FOR INVENTION]

The present disclosure to achieve the aforementioned objects is characterized by a cosmetic use of a cosmetic composition comprising a combination of at least one of serine and carnitine and at least one of allantoin and creatine for promoting skin exfoliation. Hereinafter, with reference to drawings, the present disclosure will be described in detail.

The term, "exfoliation" used herein means that the stratum corneum accumulated in the stratum corneum layer of skin is eliminated or removed from the stratum corneum layer. It is a concept that includes not only the case where it is removed by applying a physical force, but also the case where it is separated from the stratum corneum layer only by treating a cosmetic composition without applying a separate force.

Serine and carnitine means a substance which alleviates protein binding between keratinocytes through binding to calcium in skin.

Allantoin and creatine is a substance which affects the structure of the binding protein, and weakening of binding between keratinocytes may be induced through this.

According to one aspect of the present disclosure, a component for a protein structural change may be further comprised, and for example, it may be at least one selected from urea and hydroxyethyl urea.

The aforementioned substances that change protein structure have the following chemical formula in their structures in common.

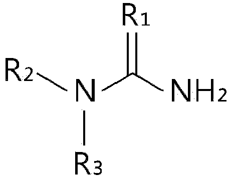

According to one aspect of the present disclosure, the cosmetic composition for skin exfoliation may further comprise a polymer material which can weaken binding between keratinocytes physicochemically.

According to one aspect of the present disclosure, the polymer substance may be at least one selected from the group consisting of cellulose nanofiber, carboxymethyl cellulose (CMC), hyaluronic acid and carbomer. Preferably, it may be cellulose nanofiber.

For serine and carnitine it was confirmed that the activity was decreased when a calcium ion was present by an exfoliation evaluation experiment and finding that calcium binding in the stratum corneum layer was the main mechanism of exfoliation, and it was confirmed that alantoin and creatine and polymer material were not affected by calcium. In addition, polymers have a unique bed structure in which the minor axis has a nanometer size and the long axis has a micrometer size, and they are mentioned in the invention of Korean Patent Publication No. 10-2017-0103698. It is expected that this unique structure may cause cracking between keratinocytes.

In other words, in order to solve the problems of skin irritation and reduced effects from increasing pH of the conventional chemical exfoliating agents showing their effects by calcium binding, the present disclosure uses materials acting in other ways, specifically, substances which weaken the binding ability between keratinocytes through protein structural changes or through physicochemical actions together, thereby achieving a synergistic effect of exfoliation.

According to one aspect of the present disclosure, the cosmetic composition for skin exfoliation may be pH 5 to 7.5, preferably, 5.5 to 7.

According to one aspect of the present disclosure, the content of the at least one of serine and carnitine may be 0.001 to 20 % by weight, preferably 0.01 to 17 % by weight, more preferably 0.05 to 15 % by weight, based on the total composition weight. When the content is less than 0.001 % by weight, it is difficult to obtain a desired effect, and when it is over 20 % by weight, the effect according to the increase of the content may be insignificant.

According to one aspect of the present disclosure, the content of the at least one of allantoin and creatinemay be 0.001 to 10 % by weight, preferably 0.01 to 5 % by weight, more preferably 0.05 to 1 % by weight, based on the total composition weight. When the content is less than 0.001 % by weight, it is difficult to obtain a desired effect, and when it is over 10 % by weight, the effect according to the increase of the content may be insignificant.

According to one aspect of the present disclosure, the content of the polymer material in the composition of the present disclosure may be 0.0001 to 5 % by weight, preferably 0.001 to 1 % by weight, more preferably 0.01 to 0.1 % by weight, based on the total composition weight. When the content is less than 0.0001 % by weight, it is difficult to obtain a desired effect, and when it is over 5 % by weight, the effect according to the increase of the content may be insignificant.

Much more specific one example of combinations exhibiting an excellent exfoliation effect even in weakly acidity and neutrality in the composition according to the present disclosure is as (A) to (H) below.

**[Table 1] (Combination A not according to the invention)**

| Combin ation | Calcium-binding substance | | | | Substance that changes protein structure | | Polymer | pH |
|---|---|---|---|---|---|---|---|---|
| | Gluconol actone (%) | Serine (%) | Carniti ne (%) | Carnosi ne (%) | Allantoi n (%) | Creatin e (%) | Cellulose nanofiber (%) | |
| A | 0.1∼15 | | | | 0.01∼0.5 | | 0.01∼0.5 | 5∼7 |
| B | | 0.01∼5 | | | 0.01∼0.5 | | | 6∼7 |
| C | | | 0.1∼10 | | 0.01∼0.5 | | | 6∼7 |
| D | | | 0.01∼5 | 0.01∼5 | 0.01∼0.5 | | | 6∼7 |
| E | | 0.01∼5 | | | | 0.01∼5 | | 6∼7 |
| F | | | 0.1∼10 | | | 0.01∼5 | | 6∼7 |
| G | | 0.01∼5 | | | 0.01∼0.5 | 0.01∼5 | | 6∼7 |
| H | | | 0.1∼10 | | 0.01∼0.5 | 0.01∼5 | | 6∼7 |

In particular, although the above combinations are applied by relatively low concentration and high pH, each relatively low exfoliation effect exhibits a synergistic effect, and they have an excellent effect in exfoliation compared to 10% gluconolactone of pH 4 ~6, and at the same time, exhibit low irritation and high efficiency.

According to one aspect of the present disclosure, the composition of the present disclosure may be applied for any kind of cosmetics, quasi drugs, and skin external application products requiring exfoliation efficacy.

Hereinafter, the present disclosure will be described in more detail by examples.

### [Experimental method]

For the porcine skin stratum corneum evaluation of the present disclosure, the exfoliation evaluation method mentioned in Journal of the European Academy of Dermatology and Venereology, 2014, 28, 415-423 was modified and used. Specifically, a sample of 6 mm diameter from 1 mm thick skin of a pig, and the like, was taken with a biopsy tool, and it was placed in a 96-well plate, and after washing it with phosphate buffered saline (PBS) once, 100 ul of the sample to be tested was added. The present sample was stored under the conditions of 37°C and 50% humidity for a day, and then the number of exfoliated stratum corneum was measured with a cell counter and a sample containing only water was plotted as a negative control group and 10% gluconolactone pH 4 was plotted as a positive control group. The result was shown in the fold increase compared to the value of the result of gluconolactone 10% pH 6 for relative comparison.

### [Example 1] Exfoliation ability by concentration and pH of gluconolactone (not according to the invention)

The exfoliation ability by concentration and pH of gluconolactone was confirmed by the aforementioned porcine skin stratum corneum evaluation. The substances and composition used in the present experiment were as the following table.

**[Table 2]**

| | Gluconolactone (%) | pH |
|---|---|---|
| Experimental group 11 | 10 | 4 |
| Experimental group 12 | 10 | 5 |
| Experimental group 13 | 10 | 6 |
| Experimental group 14 | 5 | 4 |
| Experimental group 15 | 5 | 5 |
| Experimental group 16 | 5 | 6 |
| Experimental group 17 | 2 | 4 |
| Experimental group 18 | 2 | 5 |
| Experimental group 19 | 2 | 6 |

### [Example 2] Exfoliation ability by concentration of allantoin and polymer material alone (not according to the invention)

The exfoliation ability by concentration of allantoin and polymer material alone was confirmed by the aforementioned porcine skin stratum corneum evaluation. The substances and composition used in the present experiment were as the following table.

**[Table 3]**

| | Glucono lactone (%) | Allant oin (%) | Cellulose nanofiber (%) | Carbom er (%) | Hyaluron ic acid (%) | CMC (%) | pH |
|---|---|---|---|---|---|---|---|
| Non-treated group (water) | - | - | - | - | - | - | 6 |
| Control group | 10 | - | - | - | - | - | 6 |
| Experimental group 21 | - | 0.5 | - | - | - | - | 6 |
| Experimental group 22 | - | 0.1 | - | - | - | - | 6 |
| Experimental group 23 | - | - | 0.15 | - | - | - | 6 |
| Experimental group 24 | - | - | 0.03 | - | - | - | 6 |
| Experimental group 25 | - | - | - | 0.15 | - | - | 6 |
| Experimental group 26 | - | - | - | - | 0.15 | - | 6 |
| Experimental group 27 | - | - | - | - | - | 0.15 | 6 |

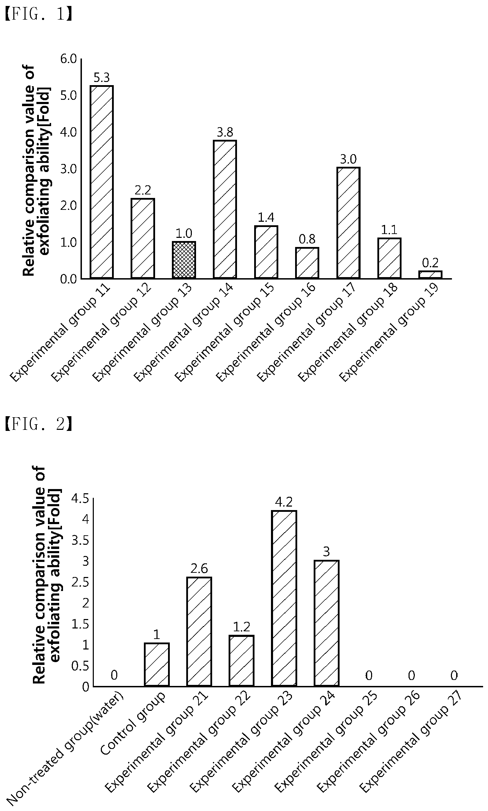

The experimental result was shown in FIG. 2 as a relative comparison value with the value of the control group as 1. Through this, it was confirmed that allantoin did not have high efficacy when used alone, and the exfoliation efficacy was not shown in each polymer except cellulose nanofiber.

### [Example 3] Exfoliation ability of combinations by concentration of gluconolactone, allantoin and cellulose nanofiber (not according to the invention)

In order to find an optimal combination of gluconolactone, allantoin and cellulose nanofiber, the exfoliation ability of combinations by concentration of them was compared through the aforementioned porcine skin exfoliation evaluation. The combination ratio used in the present experiment was as the following table.

**[Table 4]**

| | Gluconolactone (%) | Cellulose nanofiber (%) | Allantoin (%) | pH |
|---|---|---|---|---|
| Control group | 10 | - | - | 6 |
| Experimental group 31 | 10 | 0.15 | 0.5 | 6 |
| Experimental group 32 | 10 | 0.15 | 0.1 | 6 |
| Experimental group 33 | 10 | 0.03 | 0.1 | 6 |
| Experimental group 34 | 2 | 0.15 | 0.1 | 6 |
| Experimental group 35 | 2 | 0.03 | 0.1 | 6 |

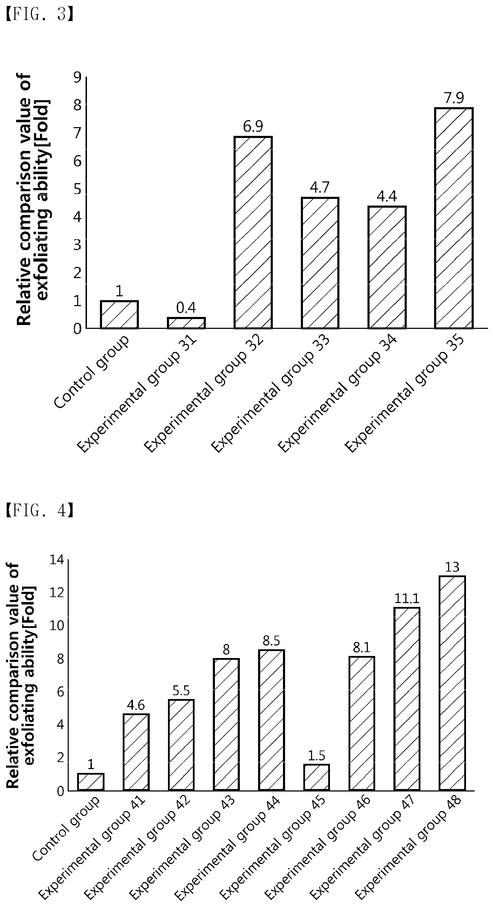

The experimental result was shown in FIG. 3. Through this, according to several times of changes of the combination ratio and pH changes, and the like, it was found that the highest exfoliation effect was shown in Experimental group 35, and this is the result exhibited as synergy of efficacy of each raw material.

### [Example 4] Exfoliation ability by concentration of carnitine, creatine and carnosine alone (not according to the invention)

The exfoliation ability by concentration of carnitine, creatine and carnosine alone was confirmed by the aforementioned porcine skin stratum corneum evaluation. The combination ratio used in the present experiment was as the following table.

**[Table 5]**

| | Gluconolactone (%) | Carnitine (%) | Creatine (%) | Carnosine (%) | pH |
|---|---|---|---|---|---|
| Control group | 10 | - | - | - | 6 |
| Experimental group 41 | - | 0.5 | - | - | 7 |
| Experimental group 42 | - | 1 | - | - | 7 |
| Experimental group 43 | - | 5 | - | - | 7 |
| Experimental group 44 | - | 10 | - | - | 7 |
| Experimental group 45 | - | - | 1 | - | 6 |
| Experimental group 46 | - | - | - | 0.2 | 7 |
| Experimental group 47 | - | - | - | 1 | 7 |
| Experimental group 48 | - | - | - | 3 | 7 |

### [Example 5] Exfoliation ability of combinations by concentration of carnitine, creatine and carnosine

In order to find an optimal combination of carnitine, creatine and carnosine, the exfoliation ability of combinations by concentration of them was compared through the aforementioned porcine skin exfoliation evaluation. The combination ratio used in the present experiment was as the following table.

**[Table 6]**

| | Glucono lactone (%) | Carnitine (%) | Allantoin (%) | Creatine (%) | Carnosine (%) | pH |
|---|---|---|---|---|---|---|
| Control group | 10 | - | - | - | - | 6 |
| Experimental group 51 | - | 0.5 | 0.1 | - | - | 6 |
| Experimental group 52 | - | 0.5 | - | 0.1 | - | 6 |
| Experimental group 53 | - | 5 | 0.1 | - | - | 6 |
| Experimental group 54 | - | 5 | 0.1 | - | - | 7 |
| Exper imental group 55 | - | 5 | - | 1 | - | 7 |
| Exper i men tal group 56 | - | 5 | 0.1 | 1 | - | 7 |
| Experimental group 57 | - | 0.5 | 0.1 | - | 0.2 | 6 |
| Experimental group 58 | - | 0.5 | 0.1 | - | 0.2 | 7 |

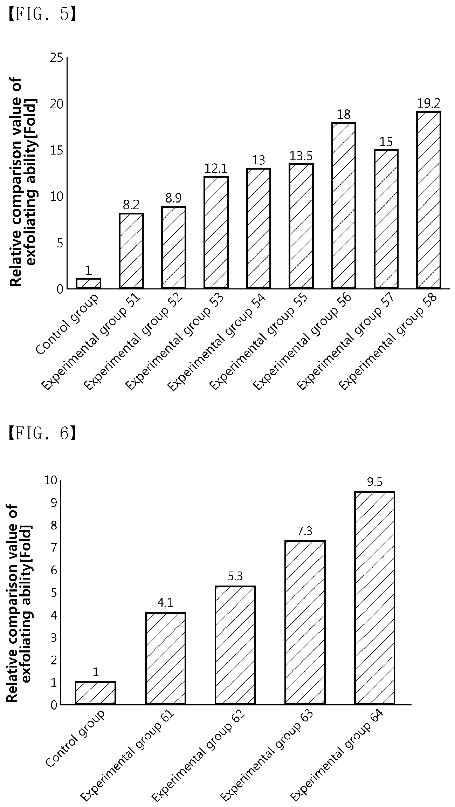

The experimental result was shown in FIG. 5. Through this, it was found that the optimization of efficiency was achieved in combinations of carnitine 0.5-5% + allantoin 0.1% pH 7, carnitine 0.5-5% + creatine 1% + allantoin 0.1% pH 6, and carnitine 0.5-5% + carnosine 0.2-3% + allantoin 0.1% pH 7. In particular, in was confirmed that Experimental groups 57 and 58 showed the efficiency higher than the highest concentration and showed a synergistic effect greater than the efficacy of each raw material, although low concentrations of carnitine and carnosine were used.

### [Example 6] Exfoliation ability by concentration of serine (not according to the invention)

The exfoliation ability by concentration of serine was confirmed by the aforementioned porcine skin stratum corneum evaluation. The combination ratio used in the present experiment was as the following table.

**[Table 7]**

| | Gluconolactone (%) | Serine (%) | pH |
|---|---|---|---|
| Control group | 10 | - | 6 |
| Experimental group 61 | - | 1 | 6 |
| Experimental group 62 | - | 2 | 6 |
| Experimental group 63 | - | 5 | 6 |
| Experimental group 64 | - | 10 | 6 |

### [Example 7] Exfoliation ability of combinations by concentration of serine, creatine and allantoin combination

The exfoliation ability of combinations by concentration of serine. creatine and allantoin was confirmed by the aforementioned porcine skin stratum corneum evaluation. The combination ratio used in the present experiment was as the following table.

**[Table 8]**

| | Gluconolacton e (%) | Serine (%) | Creatine (%) | Allantoin (%) | pH |
|---|---|---|---|---|---|
| Control group | 10 | - | - | - | 6 |
| Experimental group 71 | - | 1 | - | 0.1 | 6 |
| Exper imental | - | 1 | 1 | - | 6 |
| group 72 | | | | | |
| Experimental group 73 | - | 1 | 1 | 0.1 | 6 |

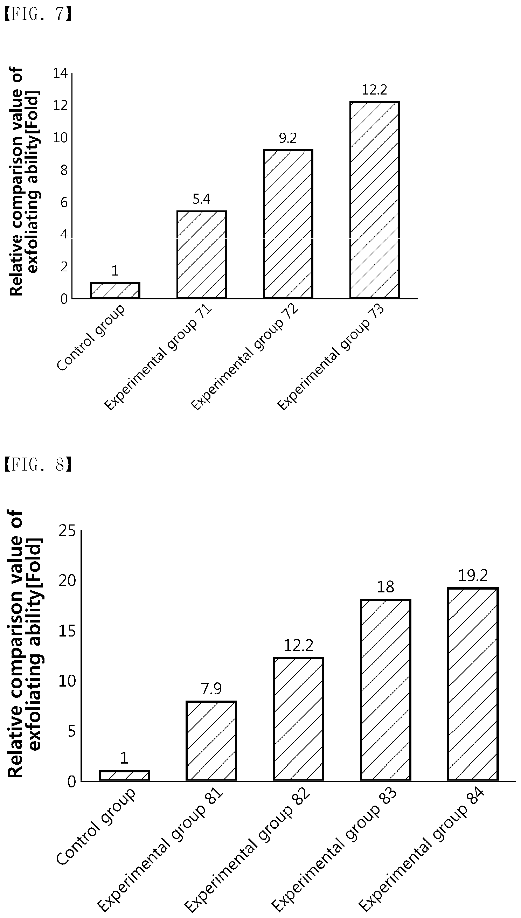

The experimental result was shown in FIG. 7. Though this, it was found that the combination optimizing the synergistic effect when combined with serine was serine 1% + creatine 1% + allantoin 0.1% pH 6.

### [Example 8] Exfoliation ability of various combinations

Based on the aforementioned experimental results, various combinations as the following table were prepared, and the exfoliation ability for time was directly compared through the aforementioned porcine skin exfoliation evaluation.

**[Table 9] (Experimental Group 81 not according to the invention)**

| | Gluconola ctone (%) | Serin e (%) | Carni tine (%) | Carno sine (%) | Allan toin (%) | Great ine (%) | Cellulose nanofiber (%) | pH |
|---|---|---|---|---|---|---|---|---|
| Control group | 10 | - | - | - | - | - | - | 6 |
| Experimen tal group 81 | 2 | - | - | - | 0.1 | - | 0.03 | 6 |
| Experimen tal group 82 | - | 1 | - | - | 0.1 | 1 | - | 6 |
| Experimen tal group 83 | - | - | 5 | - | 0.1 | 1 | - | 7 |
| Experimen tal group 84 | - | - | 0.5 | 0.2 | 0.1 | - | - | 7 |

The experimental result was shown in FIG. 8. As the experimental result, it was confirmed that all the four experimental group combinations showed an excellent exfoliation ability in weakly acidity and neutrality of pH 6 or 7, and through this, there was a combination and a ratio which showed a synergistic effect in the exfoliation ability of each raw material when combined by classification according to its property, and thereby the exfoliation ability could be maximized. In particular, it was confirmed that through combination between raw materials having a synergistic effect, it could be possible to show higher efficiency and lower side effects, which are unique side effects of raw materials that can be caused when using one raw material at a high concentration.

### [Example 9] Stratum corneum turnover enhancement evaluation by DHA staining

Subjects were 14 healthy males and females between ages of 20 to 40, and were under stratum corneum turnover enhancement evaluation by DNA staining.

Before applying a sample, the color inside the lower arm and upper arm of subjects was measured with a chroma meter, and then about 0.4 ml of dihydroxy acetone (DHA) at a concentration of 10% was attached to the inner upper and lower arm for 6 hours. After 24 hours, the color of the brown-colored area by DHA was measured to compare the color difference from before the sample was applied. Thereafter, while applying the sample twice a day, the degree of discoloration was measured with a chroma meter every day to measure the time taken to return to the original skin color. 50 % TT (Turnover Time) means the time taken for replacing 50% of the existing stratum corneum layer with a new stratum corneum layer, and in the present experiment, the time taken for skin colored by DHA to return to the original to 50% is shown by relative comparison analysis by regression analysis. The result of the negative control group was estimated as 10 days, and the hypothetical positive control group (a sample that returns to 50% in one day) was estimated as 1 day, to calculate them and compare relative values.

When the result of the non-treated group was estimated as 10 days, the result of the experiment for the following experimental groups was as the table below.

**[Table 10] (Experimental Group 91 not according to the invention)**

| | Glucono lactone (%) | Serin e (%) | Carni tine (%) | Alla ntoi n (%) | Cellulose nanofiber (%) | pH | 50%TT (day) |
|---|---|---|---|---|---|---|---|
| Control group (non-treated) | - | - | - | - | - | - | 10 |
| Experimental group 91 | 2 | - | - | 0.1 | 0.03 | 5.5 | 8.7 |
| Experimental group 92 | - | - | 5 | 0.1 | - | 7 | 8.3 |
| Experimental | - | 5 | - | 0.1 | - | 6 | 6.8 |
| group 93 | | | | | | | |
| Experimental group 94* | - | 1 | - | - 0.1 | - | 6 | 7.9 |
| Experimental group 95* | - | - | 0.5 | 0.1 | - | 7 | 7.9 |

| | | | | | | | |
|---|---|---|---|---|---|---|---|
| (* Experimental groups 94 and 95 are essence formulations) | | | | | | | |

As the experimental result, in case of Experimental group 91, 50%TT was 8.7 days, and the stratum corneum turnover date was advanced by about 1.3 days, and in case of Experimental group 92, it was 8.3 days, and it was advanced by 1.7 days, and in case of Experimental group 93, it was 6.8 days and it was advanced by 3.2 days. In addition, among combinations, when applied in an essence formulation, in case of Experimental group 94 and Experimental group 95, it was 7.9 days both, and it was advanced by 2.1 days equally. All the combinations did not cause side effects such as erythema, itching, burning feeling, and the like, during and after the experimental period.

Through this, it was confirmed that the combination of which efficacy was found by the porcine skin exfoliation evaluation could be used as a stratum corneum turnover enhancement material in clinical trials, and it was confirmed that its efficacy was exhibited when applied to formulations.

## Claims

1. A cosmetic use of a cosmetic composition comprising a combination of at least one of serine and carnitine and at least one of allantoin and creatine for promoting skin exfoliation.

2. The cosmetic use according to claim 1, wherein the cosmetic composition further comprises a polymer material.

3. The cosmetic use according to claim 2, wherein the polymer material is at least one selected from the group consisting of cellulose nanofiber, carboxymethyl cellulose (CMC), hyaluronic acid and carbomer.

4. The cosmetic use according to claim 1, wherein the cosmetic composition has pH 5 to 7.5.

5. The cosmetic use according to claim 1, wherein the content of the at least one of serine and carnitine is 0.001 to 20 % by weight based on the total composition weight.

6. The cosmetic use according to claim 1, wherein the content of the at least one of allantoin and creatine is 0.001 to 10 % by weight based on the total composition weight.

7. The cosmetic use according to claim 3, wherein the content of the polymer material is 0.0001 to 5 % by weight based on the total composition weight.

## Patentansprüche

1. Kosmetische Verwendung einer kosmetischen Zusammensetzung, umfassend eine Kombination von mindestens einem von Serin und Carnitin und mindestens einem von Allantoin und Kreatin zur Förderung der Hautexfoliation.

2. Kosmetische Verwendung nach Anspruch 1, wobei die kosmetische Zusammensetzung ferner ein Polymermaterial umfasst.

3. Kosmetische Verwendung nach Anspruch 2, wobei das Polymermaterial mindestens eines ist, ausgewählt aus der Gruppe bestehend aus Cellulose-Nanofaser, Carboxymethylcellulose (CMC), Hyaluronsäure und Carbomer.

4. Kosmetische Verwendung nach Anspruch 1, wobei die kosmetische Zusammensetzung einen pH-Wert von 5 bis 7,5 aufweist.

5. Kosmetische Verwendung nach Anspruch 1, wobei der Gehalt des mindestens einen von Serin und Carnitin 0,001 bis 20 Gew.-%, bezogen auf das Gesamtgewicht der Zusammensetzung, beträgt.

6. Kosmetische Verwendung nach Anspruch 1, wobei der Gehalt des mindestens einen von Allantoin und Kreatin 0,001 bis 10 Gew.-%, bezogen auf das Gesamtgewicht der Zusammensetzung, beträgt.

7. Kosmetische Verwendung nach Anspruch 3, wobei der Gehalt des Polymermaterials 0,0001 bis 5 Gew.-%, bezogen auf das Gesamtgewicht der Zusammensetzung, beträgt.

## Revendications

1. Utilisation à des fins cosmétiques d'une composition cosmétique comprenant une combinaison d'une au moins parmi la sérine et la carnitine et d'une au moins parmi l'allantoïne et la créatine pour favoriser l'exfoliation cutanée.

2. Utilisation à des fins cosmétiques selon la revendication 1, où la composition cosmétique comprend en outre un matériau polymère.

3. Utilisation à des fins cosmétiques selon la revendication 2, où le matériau polymère est un au moins sélectionné dans le groupe consistant en des nanofibres de cellulose, la carboxyméthylcellulose (CMC), l'acide hyaluronique et un carbomère.

4. Utilisation à des fins cosmétiques selon la revendication 1, où la composition cosmétique a un pH qui va de 5 à 7,5.

5. Utilisation à des fins cosmétiques selon la revendication 1, où la teneur en la une au moins parmi la sérine et la carnitine, rapportée au poids total de la composition, va de 0,001 à 20 % en poids.

6. Utilisation à des fins cosmétiques selon la revendication 1, où la teneur en la une au moins parmi l'allantoïne et la créatine, rapportée au poids total de la composition, va de 0,001 à 10 % en poids.

7. Utilisation à des fins cosmétiques selon la revendication 3, où la teneur en le matériau polymère, rapportée au poids total de la composition, va de 0,0001 à 5 % en poids.
